# EUROPEAN PATENT SPECIFICATION

(11) **EP 0 666 755 B1**
(45) Date of publication and mention of the grant of the patent: **23.04.2003**
(21) Application number: 93921439.1
(22) Date of filing: 09.09.1993
(51) Int. Cl.: A61K 38/16

(54) **INHIBITION OF HIV-INFECTION**
HEMMUNG VON HIV-INFEKTION
INHIBITION DE L'INFECTION PAR L'HIV

(30) Priority: 09.09.1992 US 943369
(43) Date of publication of application: 16.08.1995
(73) Proprietor: Amgen Inc., Thousand Oaks, CA 91320-1799 (US); THE GOVERNMENT OF THE UNITED STATES OF AMERICA as represented by the SECRETARY OF THE DEPARTMENT OF HEALTH AND HUMAN SERVICES, Bethesda, MD 20892-9902 (US)
(72) Inventor: EISENBERG, Stephen, Boulder, CO 80303 (US); WAHL, Sharon M., Gaithersburg, MD 20878 (US); THOMPSON, Robert C., Boulder, CO 80303 (US)
(74) Representative: Grünecker, Kinkeldey, Stockmair & Schwanhäusser Anwaltssozietät
(86) International application number: US9308486
(87) International publication number: WO94006454

(56) References cited:
- EP-A- 0 346 500
- EP-A- 0 384 559
- WO-A-91/02540
- WO-A-91/08769
- WO-A-92/21373
- NUCLEIC ACIDS RESEARCH. vol. 14, no. 20 , 1986 , ARLINGTON, VIRGINIA US pages 7883 - 7896 G. STETLER ET AL. 'Isolation and sequence of a human gene encoding a potent inhibitor of leukocyte proteases'
- TRENDS IN PHARMACEUTICAL SCIENCES vol. 10, no. 8 , 1989 pages 305 - 307 M. JOHNSTON ET AL 'HIV proteinase as a target for drug action'
- FEBS LETTERS. vol. 247, no. 1 , April 1989 , AMSTERDAM NL pages 113 - 117 A. RICHARDS ET AL 'Effective blocking of HIV-1 proteinase activity by characteristic inhibitors of aspartic proteinases'
- PATENT ABSTRACTS OF JAPAN vol. 14, no. 390 (C-751)(4333) 23 August 1990 & JP,A,02 145 527 (NIPPON CHEM RES K.K.) 5 June 1990
- JOURNAL OF BIOLOGICAL CHEMISTRY. vol. 268, no. 20 , 15 July 1993 , BALTIMORE US pages 14583 - 14585 W. LU ET AL 'Arg15-lys17-arg18 turkey ovomucoid third domain inhibits human furin'
- FEBS LETTERS. vol. 317, no. 1,2 , February 1993 , AMSTERDAM NL pages 167 - 172 L-E AVRIL ET AL 'Interaction between a membrane-associated serine proteinase of U-937 monocytes and peptides from the V3 loop of the human immunodeficiency virus type 1 (HIV-1) gp120 envelope glycoprotein'
- NATURE. vol. 360 , 26 November 1992 , LONDON GB pages 358 - 361 S. HALLENBERGER ET AL 'Inhibition of furin-mediated cleavage activation of HIV-1 glycoprotein'

## Description

This invention relates to the field of the treatment of retroviral infections and, more particularly, to the treatment of human immunodeficiency virus (HIV) infection and associated disease, including acquired immune deficiency syndrome (AIDS).

### Background of the Invention

Retroviral agents have been implicated in a number of diseases, including cancer, autoimmune disease and AIDS. Human immunodeficiency virus (HIV) infection causes chronic progressive depletion of CD4⁺ T lymphocytes (CD4⁺ cells) and infection of macrophages, resulting in acquired immune deficiency syndrome. Currently zidovudine (AZT), an analogue of thymidine, is the primary anti-viral drug used in the treatment of HIV infection, although two other agents with a similar mechanism of action, dideoxyinosine (ddI) and dideoxycytosine (ddC), are also used. Colley, T.P. et al., New Engl. J. Med. (1990) 322:1340-45; Fischl, M.A., et al., New Engl. J. Med. (1987) 317:185-91. These agents are effective in inhibiting viral replication, and can stabilize the CD4⁺ cell levels, but they are unable to eliminate one of the major viral reservoirs, HIV infected macrophages. Gartner, S., et al., Science (1986) 233:215-19. Severe toxicity, particularly involving HIV host bone marrow is also associated with higher doses of AZT treatment, and the beneficial effects of the drug in AIDS patients diminishes after prolonged therapy; HIV strains resistant to AZT also have been observed in treated patients. These findings have prompted the search for alternative drugs for the treatment of HIV infection, particularly agents with a different mechanism of action.

Human immunodeficiency virus type 1 (HIV-1), a retrovirus, is the etiologic cause of AIDS. The HIV-1 envelope glycoprotein, gp120, specifically binds to the CD4 receptor on T lymphocytes and on monocytes and macrophages. Although infection of T lymphocytes requires cellular proliferation and DNA synthesis, productive infection of monocytes can occur independently of cellular DNA synthesis (Weinberg, J.B., et al,. (1991) J. Exp. Med. 174:1477-82). When HIV-1 infects activated CD4⁺ lymphocytes, it is lethal, but infected monocytes are relatively resistant to destruction by the virus. Consequently, these cells, once infected with HIV-1, serve as long-lived reservoirs of the virus. Not only are these cells a source of replicating virus, but their virally-mediated dysfunction may contribute to increased susceptibility to opportunistic infections that are the hallmark of AIDS.

Because monocyte-macrophages serve as reservoirs for HIV-1, selective targeting of this population, in addition to T lymphocytes, warrants further consideration (Finberg, R.W., et al., Science 252:1703-05, 1991. Early reports from Fox's group (JADA 118:709-711, 1989) indicated that a component of human saliva blocks HIV replication. More recently, Hattori (FEBS Lett. 248:48-52, 1989) showed that an inhibitor of tryptase (a trypsin-like enzyme) can inhibit syncytia formation of T-cells induced by HIV.

In exploring various potential modulators of HIV-1 infection, we have recently identified an endogenous source of inhibitory activity which retards HIV-1 infection and/or replication.

The factor responsible for the antiviral activity is serine leukocyte protease inhibitor (SLPI). SLPI is a potent inhibitor of human leukocyte elastase and cathepsin G and of human trypsin, and has been purified from parotid secretions (Thompson, R.C. and K. Ohlsson, (1986) Proc. Natl. Acad. Sci. USA, 83:6692-96; and U.S. Patent No. 4,760,130, both of which are incorporated herein by reference). SLPI is now available through production by recombinant DNA techniques; U.S. patent application No. 07/712,354, filed June 7, 1991, PCT application No. WO86/03519, filed December 4, 1985, and European patent application 85 905 953.7, filed December 4, 1985, each of which are incorporated herein by reference).

The ability of SLPI and/or its derivatives and analogs to block HIV-1 infection and/or replication can provide the basis for therapeutic intervention in HIV-1 infection.

### Summary of the Invention

The present invention provides the use of serine leukocyte protease inhibitor (SLPI) or SLPI mutein for preparing a medicament for inhibiting human immunodeficiency virus (HIV) infection.

### Brief Description of the Figures

Figure 1. SLPI blocks HIV replication in monocytes in a dose-dependent manner. Elutriated human monocytes were plated and exposed to HIV ± SLPI for one hour at 37°C, washed, and incubated at 37°C, drawing off supernatants and adding fresh medium every four days. The EC₅₀ for this experiment was <0.1 µg/ml (8.5 nM) with complete inhibition at 10 µg/ml (850 nM).

Figure 2. The SLPI inhibitory effect is long-lasting. At the 18-day time point, HIV is still 90% inhibited.

### Detailed Description of the Invention

The present invention provides methods for preventing HIV infection of mammalian cells, particularly human cells, and associated diseases, including acquired immune deficiency syndrome (AIDS).

The term "pharmaceutically acceptable carrier" as used herein means a non-toxic, generally inert vehicle for the active ingredient, which does not adversely affect the ingredient or the patient to whom the formulation is administered.

The term "effective amount" as used herein means a predetermined amount of SLPI, or an analog or derivative thereof, sufficient to be effective against HIV in vivo.

Retroviral infections are treated by administering anti-retroviral agents in doses sufficient to diminish the effects of such infection. Retroviral infections are implicated in a number of diseases, including but not limited to cancer, autoimmune disease, and acquired immune deficiency syndrome. Human immunodeficiency virus infection is of particular interest according to the present invention.

A variety of anti-retroviral agents are known in the art. Most of these inhibit the activity of retroviral reverse transcriptase and include zidovudine (AZT), an analogue of thymidine, dideoxyinosine (ddI), and dideoxycytosine (ddC). Zidovudine is the primary anti-viral drug used in the treatment of HIV infection. Anti-retroviral agents are generally efficacious in a dose ranging from about 50 mg/day to about 1000 mg/day, more particularly from about 100 mg/day to about 500 mg/day, and in the case of zidovudine, specifically about 300 mg/day to about 500 mg/day. These agents are generally administered in oral formulations.

The protease inhibitors used in this invention can be prepared by means well known to those skilled in the art (see, e.g., U.S. Patent No. 4,760,130; European patent application 85 905 953.7, PCT application WO86/03519, and U.S. patent application 07/712,354, supra).

The serine protease inhibitors of the present invention are single-polypeptide-chain proteins which are substantially homologous to, and biologically equivalent to, the native serine protease inhibitor isolated from human parotid secretions. The native serine protease inhibitor is also referred to as the native parotid inhibitor. By "biologically equivalent" as used throughout the specification and claims, is meant that the compositions are capable of inhibiting the monocyte-derived protease that is inhibited by SLPI, but not necessarily to the same degree. By "derivatives" as used throughout the ensuing specification and claims, is meant a degree of amino acid homology to the native parotid inhibitor, preferably in excess of 40%, most preferably in excess of 50%, with a particularly preferred group of proteins being in excess of 60% homologous with the native parotid inhibitor. The percentage homology, as above described, is calculated as the percentage of the components found in the smaller of the two sequences that may also be found in the larger of the two sequences, a component being understood as a sequence of four, contiguous amino acids.

One useful SLPI derivative is CLPI, a truncated SLPI molecule having only the last 60 amino acids of the native parotid inhibitor. These 60 amino acids are:

Leu Asp Pro Val Asp Thr Pro Asn Pro Thr Arg Arg Lys
Pro Gly Lys Cys Pro Val Thr Tyr Gly Gln Cys Leu Met
Leu Asn Pro Pro Asn Phe Cys Glu Met Asp Gly Gln Cys
Lys Arg Asp Leu Lys Cys Cys Met Gly Met Cys Gly Lys
Ser Cys Val Ser Pro Val Lys Ala.

The following nucleotide sequence has been used to encode the above 60 amino acid molecule:

CTG GAT CCT GTT GAC ACC CCA ACA CCA ACA AGG AGG AAG
CCT GGG AAG TGC CCA GTG ACT TAT GGC CAA TGT TTG ATG
CCT AAC CCC CCC AAT TTC TGT GAG ATG GAT GGC CAG TGC
AAG CGT GAC TTG AAG TGT TGC ATG GGC ATG TGT GGG AAA
TCC TGC GTT TCC CCT GTG AAA GCT.

CLPI has been constructed by deleting from the SLPI gene the signal sequence and the nucleotides corresponding to the first 47 amino acids of the mature SLPI protein as described in U.S. patent application 07/712,354. CLPI can also be made by the method of Example 8 described in both PCT application WO86/03519 and European patent application 85 905 953.7. Although Example 8 in these two applications recites a method of making SLPI, this method can also be used to make CLPI. CLPI can be used to generate antibodies useful in purifying SLPI. Antibodies can be produced, for example, by the methods discussed in E. Harlow & D. Lane, Antibodies: A Laboratory Manual, pp. 92-114 (Cold Springs Harbor Laboratory, 1988).

Also provided is the use of specific SLPI muteins that have at least equivalent, and in some cases, greater activity than the native protein. Particularly useful SLPI muteins include substitution of the following amino acids at the residue position enumerated: Gly 20, Gly 72, Val 72, and Phe 72.

CLPI muteins are also within the scope of the invention. CLPI muteins which correspond to the SLPI muteins Gly 72, Val 72, and Phe 72 are herein referred to as Gly 25, Val 25, and Phe 25. Some contemplated CLPI muteins have the following amino acid sequence: 
Leu Asp Pro Val Asp Thr Pro Asn Pro Thr Arg Arg Lys
Pro Gly Lys Cys Pro Val Thr Tyr Gly Gln Cys R8 R3
R9 Asn Pro Pro Asn Phe Cys Glu R4 Asp Gly Gln Cys
Lys Arg Asp Leu Lys Cys Cys R5 Gly R6 Cys Gly Lys
Ser Cys Val Ser Pro Val Lys R7
 wherein R7 is alanine, and R3, R4, R5, R6, and R8 are the same or different amino acids and one or more of R3, R4, R5, R6, and R8 may be methionine, valine, alanine, phenylalanine, tyrosine, tryptophan, lysine, glycine, or arginine. Also included are for example, PEGylated forms of SLPI which may have improved therapeutic characteristics over the native SLPI protein. Muteins which may be suitable for PEGylation include those having a cysteine residue at positions 13, 23, 52, 58, 68, and/or 75 of SLPI and at the corresponding sites 5, 11, 21, and 28 in CLPI. Preparation of cysteine muteins for PEGylation is described in PCT application WO 92/16221, filed March 13, 1992, which is specifically incorporated herein by reference. A useful step in mutein production can include a refolding step in which cysteine is added to the solution containing the protein. The cysteine can aid in refolding and can bond to the substituted free cysteine in the mutein. One may also isolate from monocytes the SLPI inhibitable protein (SIP) from human monocyte cells using standard biochemical techniques well known to those skilled in the art and purify proteins having proteolytic activity which is inhibited by SLPI. After purifying the protein (and, if necessary, sequencing it, cloning its gene, and expressing it in host cells, i.e., recombinantly producing SIP), one can screen for inhibitors of SIP by means well known to those skilled in the art. Alternatively, one can determine its structure and design inhibitors therefrom, also by means well known to those skilled, in the art.

When SLPI, or mutein thereof, is used to combat HIV infections in a human, the compound can be administered orally or parenterally, in a vehicle comprising one or more pharmaceutically acceptable carriers, the proportion of which is determined by the solubility and chemical nature of the compound, chosen route of administration and standard biological practice. For oral administration, SLPI, or analog or derivative thereof, can be formulated in unit dosage forms such as capsules or tablets each containing a predetermined amount of the active ingredient, ranging from about 10 to 1000 mg, more preferably 10-200 mg per day per patient, even more preferably 20-200 mg per day per patient, in a pharmaceutically acceptable carrier.

For parenteral administration, the SLPI or a mutein thereof, is administered by either intravenous, subcutaneous or intramuscular injection, in compositions with pharmaceutically acceptable vehicles or carriers. For administration by injection, it is preferred to use the compound in solution in a sterile aqueous vehicle which may also contain other solutes such as buffers or preservatives as well as sufficient quantities of pharmaceutically acceptable salts or of glucose to make the solution isotonic. Subcutaneous injection is the preferred route of administration. Dosages are essentially the same as those set forth above for oral administration.

Suitable vehicles or carriers for the above noted formulations can be found in standard pharmaceutical texts, e.g., in "Remington's Pharmaceutical Sciences," 16th ed, Mack Publishing Company, Easton, PA, 1980, and incorporated herein by reference.

The dosage of the compound will vary with the form of administration and the particular active agent chosen. Furthermore, it will vary with the particular patient or host (including mammals, including humans) under treatment. Generally, treatment is initiated with small dosages substantially less than the optimum dose of the compound. Thereafter, the dosage is increased by small increments until the optimum effect under the circumstances is reached. In general, the compound is most desirably administered at a concentration level that will generally afford antivirally effective results without causing any harmful or deleterious side effects. It is desirable to maintain a blood level of the compound at a level sufficient to inhibit retrovirus infection of the host cell. This can be estimated by assaying the amount of compound that is effective in preventing retroviral infection of host cells, e.g., HIV into monocytes, in vitro, and then, using standard pharmacokinetic techniques, determining the amount of compound required to keep plasma level at the same inhibitory level, or up to 10-100 times more.

Although the formulations disclosed hereinabove are effective and relatively safe medications for treating HIV infections, the possible concurrent administration of these formulations with other antiviral medications or agents to obtain beneficial results is not excluded. Such other antiviral medications or agents include soluble CD4, zidovudine, dideoxycytidine, phosphonoformate, ribavarin, antiviral interferons (e.g. alpha -interferon or interleukin-2) or aerosol pentamidine.

The invention is exemplified by the following illustrative examples:

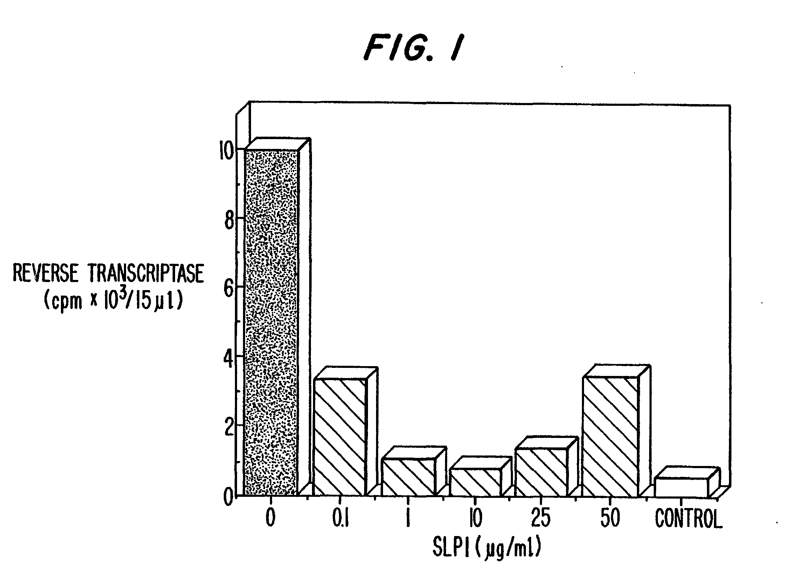

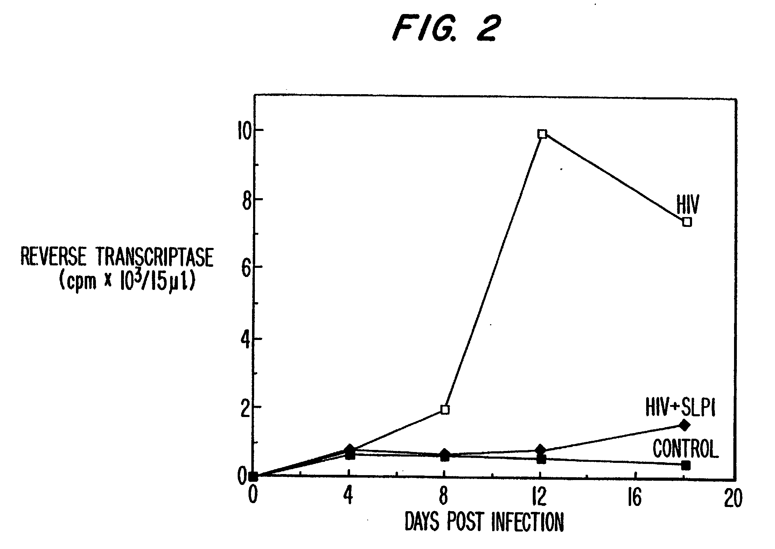

Example 1. Peripheral blood monocytes (PBM) were isolated from healthy donors by elutriation, plated in culture dishes, and incubated for several days. SLPI was mixed with HIV (Bal) and applied to PBM for one hour at 37°C. Cells were washed and incubated for additional time, with media changes and reverse transcriptase determinations on supernatants done every three days. We found that SLPI effectively blocks HIV replication at a concentration of 1 µg/ml (Figure 1). At concentrations ≤ 20 µg/ml, SLPI inhibition is diminished. The inhibitory effect is long lasting, with significant inhibition seen out to 18 days (Figure 2).

Example 2. PBM were plated and incubated as in Example 1. SLPI was applied to cells for about one hour, cells were then washed, and treated with HIV. Medium was changed and assays done as in Example 1. We found that SLPI was more effective at blocking HIV when cells were pretreated with SLPI than when cells were treated with a mix of SLPI and HIV.

Example 3. We have also demonstrated using essentially the same protocol as in Example 1, but substituting T-cells for monocytes, that SLPI is effective in inhibiting HIV replication in T-cells.

Example 4. A human T-lymphocytic cell line (H-9) was maintained in suspension culture in RPMI 1640 with 10% fetal calf serum (FCS) and 200 micrograms per liter gentamicin. SLPI was added to the culture medium at a final concentration of 100 micrograms per milliliter. After 24 hours, cells were washed, inoculated for four hours with HIV strain IIIB, washed again, and resuspended at a density of 500,000 cells per milliliter. Media was supplemented and maintained with SLPI at a final concentration of 100 micrograms per milliliter immediately after resuspension (T=0) or 2 days after resuspension (T=2). Culture supernatant was collected and cultures were fed every 2 days. Supernatant collected 8 days after infection was assayed for reverse transcriptase activity by measuring uptake of tritiated thymidine onto a poly(rA)-oligo(dT) template.

As shown in Table 1, in SLPI pretreated cells, SLPI inhibited viral replication by approximately 62% and 54% when added immediately after infection and 2 days after infection, respectively.

**TABLE 1**

| **SLPI PRE-TREATED CELLS** | | | | |
|---|---|---|---|---|
| | **Negative Control** | **Positive Control** | **T = 0** | **T = 2** |
| **RT Activity** **(mean cpm)** | 955 | 86,205 | 32,594 | 39,554 |
| **Standard Deviation** | ± 330 | ± 9,676 | ± 7,220 | ± 8,737 |

Example 5. The experiment was performed as described in Example 4 except that 1000-fold concentrated HIV strain IIIB was incubated with 100 micrograms per milliliter SLPI for 6 hours on ice prior to inoculation. This HIV/SLPI mixture was diluted 1000-fold prior to the four hour inoculation.

As shown in Table 2, using SLPI pre-treated virus and cells, SLPI inhibited viral replication by approximately 64% and 26% when added immediately after infection and 2 days after infection, respectively.

**TABLE 2**

| **SLPI PRE-TREATED VIRUS AND CELLS** | | | | |
|---|---|---|---|---|
| | **Negative Control** | **Positive Control** | **T = 0** | **T = 2** |
| **RT Activity** **(mean cpm)** | 2,889 | 59,004 | 20,676 | 43,432 |
| **Standard Deviation** | ± 565 | ± 10,988 | ± 4,111 | ± 14,982 |

Example 6. The experiment was performed as in Example 5 except that cells were clean, i.e. not cultured with SLPI prior to inoculation. Using clean cells and SLPI pre-treated virus, SLPI inhibited viral replication by approximately 59% and 32% when added immediately after infection and 2 days after infection, respectively (Table 3).

**TABLE 3**

| **SLPI PRE-TREATED VIRUS** | | | | |
|---|---|---|---|---|
| | **Negative Control** | **Positive Control** | **T = 0** | **T = 2** |
| **RT Activity** **(mean cpm)** | 4,763 | 70,076 | 28,383 | 47,436 |
| **Standard Deviation** | ± 1,698 | ± 15,803 | ± 5,520 | ± 11,679 |

Example 7. The experiment was performed as in Examples 4-6 except that neither cells nor virus were exposed to SLPI prior to inoculation. Using clean cells and clean virus, SLPI inhibited viral replication by approximately 50% and 42% when added immediately after infection and 2 days after infection, respectively (Table 4). Table 5 shows the reverse transcriptase activity which was present in culture supernatant assayed 4, 6, and 8 days after infection.

**TABLE 4**

| **CLEAN CELLS AND VIRUS** | | | | |
|---|---|---|---|---|
| | **Negative Control** | **Positive Control** | **T = 0** | **T = 2** |
| **RT Activity** **(mean cpm)** | 531 | 79,356 | 38,969 | 46,004 |
| **Standard Deviation** | ± 186 | ± 17,497 | ± 7,700 | ± 8,492 |

**TABLE 5**

| **CLEAN CELLS AND VIRUS** | | | | |
|---|---|---|---|---|
| | **Negative** | **Positive** | **T = 0** | **T = 2** |
| **Day 4 (mean cpm)** | 435 | 1,556 | 797 | 1,287 |
| **Standard Deviation** | ± 85 | ± 300 | ± 222 | ± 204 |
| **Day 6 (mean cpm)** | 952 | 72,085 | 15,846 | 41,240 |
| **Standard Deviation** | ± 715 | ± 12,219 | ± 5,644 | ± 14,542 |
| **Day 8 (mean cpm)** | 1,519 | 13,853 | 7,617 | 11,946 |
| **Standard Deviation** | ± 475 | ± 3,458 | ± 3,031 | ± 2,889 |

Example 8. The effect of different SLPI muteins on viral replication was also investigated. Clean H-9 cells were incubated with clean virus for 4 hours as in Example 7. After washing, cells were resuspended at a density of 500,000 cells per milliliter in media containing 30 micrograms per milliliter SLPI or the SLPI muteins shown in Table 6. Culture supernatant was assayed for reverse transcriptase activity 8 days later (Table 6).

**TABLE 6**

| | **Negative Control** | **Positive Control** | **Wild Type** | **Gly 20** | **Gly 72** | **Val 72** | **Lys 72** | **Phe 72** |
|---|---|---|---|---|---|---|---|---|
| **RT Activity** **(mean cpm)** | 4,815 | 55,126 | 39,323 | 39,387 | 40,549 | 36,077 | 52,239 | 8,384 |
| **Standard** **Deviation** | ± 2,849 | ± 6,637 | ±10,933 | ±11,143 | ± 3,537 | ± 7,859 | ± 5,863 | ±1,924 |

Example 9. The experiment was performed as in Example 8 except that after inoculation, cells were resuspended in media containing 100 micrograms per milliliter SLPI or the Phe 72 mutein. Culture supernatant was assayed for reverse transcriptase activity 2, 4, 6, 8, and 10 days post-infection (Table 7). Tables 6 and 7 show that the effect of the Phe-72 mutein was particularly pronounced.

**TABLE 7**

| | **Negative** | **Positive** | **SLPI** | **PHE-72** |
|---|---|---|---|---|
| **Day 2 (mean cpm)** | | 1,386 | 995 | 897 |
| **Standard Deviation** | | ± 914 | ± 246 | ± 472 |
| **Day 4 (mean cpm)** | | 1,356 | 1,087 | 1,380 |
| **Standard Deviation** | | ± 370 | ± 414 | ± 442 |
| **Day 6 (mean cpm)** | 1,142 | 2,103 | 1,526 | 748 |
| **Standard Deviation** | ± 389 | ± 498 | ± 508 | ± 243 |
| **Day 8 (mean cpm)** | | 77,931 | 25,241 | 3,491 |
| **Standard Deviation** | | ± 9,779 | ± 8,399 | ± 1,086 |
| **Day 10 (mean cpm)** | | 21,431 | 12,499 | 2,239 |
| **Standard Deviation** | | ± 1,890 | ± 3,495 | ± 444 |

Example 10. To determine the effect of SLPI alone, H-9 cell proliferation was evaluated by thymidine incorporation assays using 200,000 H-9 cells cultured with 100 micrograms per milliliter SLPI and without SLPI. Cultures were pulsed with media containing 2.5 microcuries of tritiated thymidine at day 0, 1, and 2; incorporated counts were measured on day 1, 2, and 3. As shown in Table 8, SLPI is not toxic to these cells.

**TABLE 8**

| **RT ACTIVITY (mean cpm) H-9 PROLIFERATION** | | | |
|---|---|---|---|
| | **Day 1** | **Day 2** | **Day 3** |
| **Control (- SLPT)** | 20,860 | 67,401 | 53,326 |
| **Standard Deviation** | ± 581 | ± 2,529 | ± 3,783 |
| **+ SLPI 100 µg/ml** | 20,437 | 61,892 | 54,592 |
| **Standard Deviation** | ± 1,503 | ± 216 | ± 2,781 |

Example 11. We also investigated inhibition of viral production from chronically infected cells using the promonocytic cell line U1. Suspension cultures of U1 were maintained in RPMI with 10% FCS and 200 micrograms per liter gentamicin. Cells were harvested, washed, and suspended at a density of 2.5 million cells per milliliter. Suspended cells were cultured overnight in media containing 100 or 200 micrograms per milliliter SLPI or media alone. Virus was induced by addition of 13-phorbol-12-myristate acetate (PMA) to a final concentration of 1 micromolar. After 48 hours, cell culture supernatant was assayed for reverse transcriptase activity as in Examples 4-9. As shown in Table 9, SLPI significantly inhibited viral production from these chronically infected cells.

**TABLE 9**

| | **- PMA** **- SLPI** | **- PMA** **+ SLPI** **(200 µg/ml)** | **+ PMA** **- SLPI** | **+ PMA** **+ SLPI** **(200 µg/ml)** | **+ PMA** **+ SLPI** **(100 µg/ml)** |
|---|---|---|---|---|---|
| **RT Activity** **(mean cpm)** | 1,052 | 994 | 5,052 | 2,864 | 2,648 |
| **Standard** **Deviation** | **±** 352 | ± 447 | ±2,053 | ± 403 | ± 374 |

The foregoing description of the invention is exemplary for purposes of illustration and explanation. It should be understood that various modifications can be made without departing from the spirit and scope of the invention. Accordingly, the following claims are intended to be interpreted to embrace all such modifications.

## Claims

1. Use of a serine leukocyte protease inhibitor (SLPI) or SLPI mutein for preparing a medicament for inhibiting human immunodeficiency virus (HIV) inflection.

2. The use according to claim 1, wherein the HIV is HIV-1.

3. The use of claim 1, wherein SLPI is administered intraperitoneally.

4. The use of claim 1, wherein SLPI is administered intravenously.

5. The use of claim 1, wherein SLPI is administered subcutaneously.

6. The use according to claim 1, wherein the SLPI mutein is the SLPI Phe 72.

7. The use according to claim 1, wherein the SLPI mutein is the SLPI Gly 20.

8. The use according to claim 1, wherein the SLPI mutein is the SLPI Gly 72.

9. The use according to claim 1, wherein the SLPI mutein is the SLPI Val 72.

10. The use according to claim 1, wherein the SLPI mutein is the CLPI Phe 25.

11. The use according to claim 1, wherein the SLPI mutein is the CLPI. Gly 25.

12. The use according to claim 1, wherein the SLPI mutein is the CLPI Val 25.

## Patentansprüche

1. Verwendung eines Serinleukozytenproteaseinhibitors (SLPI) oder SLPI-Muteins zur Herstellung eines Medikaments zur Hemmung einer Human Immunschwäche-Virus (HIV)-Infektion.

2. Die Verwendung gemäß Anspruch 1, worin das HIV HIV-1 ist.

3. Die Verwendung gemäß Anspruch 1, worin der SLPI intraperitoneal verabreicht wird.

4. Die Verwendung gemäß Anspruch 1, worin der SLPI intravenös verabreicht wird.

5. Die Verwendung gemäß Anspruch 1, worin der SLPI subkutan verabreicht wird.

6. Die Verwendung gemäß Anspruch 1, worin das SLPI-Mutein das SLPI Phe 72 ist.

7. Die Verwendung gemäß Anspruch 1, worin das SLPI-Mutein das SLPI Gly 20 ist.

8. Die Verwendung gemäß Anspruch 1, worin das SLPI-Mutein das SLPI Gly 72 ist.

9. Die Verwendung gemäß Anspruch 1, worin das SLPI-Mutein das SLPI Val 72 ist.

10. Die Verwendung gemäß Anspruch 1, worin das SLPI-Mutein das CLPI Phe 25 ist.

11. Die Verwendung gemäß Anspruch 1, worin das SLPI-Mutein das CLPI Gly 25 ist.

12. Die Verwendung gemäß Anspruch 1, worin das SLPI-Mutein das CLPI Val 25 ist.

## Revendications

1. Utilisation d'un inhibiteur de la protéase à sérine des leucocytes (SLPI) ou d'une mutéine de SLPI pour préparer un médicament pour l'inhibition de l'infection par le virus de l'immunodéficience humaine (VIH).

2. Utilisation selon la revendication 1, où le VIH est le VIH-1.

3. Utilisation selon la revendication 1, où le SLPI est administré par voie intrapéritonéale.

4. Utilisation selon la revendication 1, où le SLPI est administré par voie intraveineuse.

5. Utilisation selon la revendication 1, où le SLPI est administré par voie sous-cutanée.

6. Utilisation selon la revendication 1, où la mutéine du SLPI est le SLPI Phe 72.

7. Utilisation selon la revendication 1, où la mutéine du SLPI est le SLPI Gly 20.

8. Utilisation selon la revendication 1, où la mutéine du SLPI est le SLPI Gly 72.

9. Utilisation selon la revendication 1, où la mutéine du SLPI est le SLPI Val 72.

10. Utilisation selon la revendication 1, où la mutéine du SLPI est le CLPI Phe 25.

11. Utilisation selon la revendication 1, où la mutéine du SLPI est le CLPI Gly 25.

12. Utilisation selon la revendication 1, où la mutéine du SLPI est le CLPI Val 25.
